# EUROPÄISCHE PATENTSCHRIFT

(11) **EP 3 638 093 B1**
(45) Veröffentlichungstag und Bekanntmachung des Hinweises auf die Patenterteilung: **29.07.2026**
(21) Anmeldenummer: 18733543.5
(22) Anmeldetag: 14.06.2018
(51) Int. Cl.: A61B 1/00, A61B 1/12, A61B 1/253

(54) **FENSTERSYSTEM FÜR EINEN INTRAORALSCANNER**
WINDOW SYSTEM FOR AN INTRAORAL SCANNER
SYSTÈME DE FENÊTRE POUR UN SCANNER INTRA-ORAL

(30) Priorität: 14.06.2017 DE 102017209999
(43) Veröffentlichungstag der Anmeldung: 22.04.2020
(73) Patentinhaber: Sirona Dental Systems GmbH, 64625 Bensheim (DE)
(72) Erfinder: KIM, Hong-Keun, 60323 Frankfurt (DE); WOHANKA, Martin, 64283 Darmstadt (DE); KLEIN, Konrad, 69121 Heidelberg (DE)
(74) Vertreter: Aldridge, Henry Alexander
(86) Internationale Anmeldenummer: PCT/EP2018/065880
(87) Internationale Veröffentlichungsnummer: WO 2018/229225

(56) Entgegenhaltungen:
- DE-A1- 102009 013 615
- DE-U1- 202010 017 255
- JP-A- H03 118 021
- US-A- 5 536 236
- US-A1- 2015 238 072

## Beschreibung

### Technisches Gebiet

Die vorliegende Erfindung betrifft ein Fenstersystem für einen Intraoralscanner. Weiterhin betrifft die Erfindung einen Intraoralscanner, welcher das Fenstersystem aufweist.

### Stand der Technik

Bei intraoral bzw. intrakorporal angewendeten medizintechnischen Geräten wird durch den Einsatz eines Gehäuses eine Abgrenzung zwischen der funktionellen Einheit und dem Patienten geschaffen. Hierdurch wird unter anderem eine Verunreinigung oder Kontamination von Bauteilen innerhalb des Gehäuses verhindert. Das Gehäuse dient jedoch auch dem Schutz des Anwenders und des Patienten vor mechanischen, elektrischen, biologischen und chemischen Gefahren. Durch das Gehäuse darf die medizintechnische Funktion nicht beeinträchtigt werden. Deshalb wird bei optischen Diagnosegeräten und in der Therapie eingesetzten optischen Instrumenten der für die Funktion relevante Strahlengang durch Öffnungen transparenter Fenster oder transluzenter Fenster gewährleistet.

Das Gehäuse und dessen Teile müssen bei einfacher oder mehrfacher Anwendung hohen Anforderungen bezüglich der Funktion und Hygiene genügen, dabei jedoch gleichzeitig wirtschaftlich und alltagstauglich sein. Für eine wiederholte hygienische Anwendbarkeit muss der Kontaktbereich zwischen Patient und Gerät zu reinigen und desinfizierbar oder gegebenenfalls sterilisierbar sein.

Bei intraoral angewendeten 3D-Scannern, durch welche die Zahntopologie vermessen werden kann, führt die Unterschreitung der Oberflächentemperatur eines transparenten Fensters unter den Taupunkt bei Kontakt mit ausgeatmeter Atemluft des Patienten oder der Luft im Mundinnenraum zu Beschlag. Die Bildung des Kondensats verändert den Lichtweg. Im Falle einer 3-dimensionalen Vermessung durch z. B. Streifenprojektion, Triangulation oder Konfokalmessung kann dies zu signifikanten fehlerhaften Daten und/oder zu einer zu geringen Datendichte führen.

Das Beschlagen eines transparenten Fensters kann durch die Zufuhr von kalter oder warmer Luft vermieden werden. Der Luftstrom kann bei sensiblen Patienten jedoch zu Unbehagen führen. Des Weiteren führt die Erzeugung eines Luftstroms zu einem erhöhten Stromverbrauch und zu Geräuschemissionen. Außerdem nimmt die hierfür erforderliche Düse Platz am Intraoralscanner ein.

Aus der DE 10 2009 013 615 A1 ist ein zahnmedizinisches Diagnosegerät mit Bilderfassungsmitteln bekannt, welches ein im Strahlengang der Mittel zur Bilderfassung befindliches Fenster aufweist. Das Fenster besteht aus Quarzglas, Saphir oder einem Kunststoff. Zur Beheizung des Fensters ist eine Widerstandsheizung vorgesehen.

Die beschreibt einen intraoral angewendeten 3D-Scanner, dessen Fenster mit einem Heizer verbunden ist. Der Heizer weist eine elektromagnetische Wärmequelle auf.

Es ist eine Aufgabe der vorliegenden Erfindung, ein Fenstersystem für einen Intraoralscanner sowie einen damit versehenen Intraoralscanner bereitzustellen, welches auch ohne Erzeugung eines Luftstroms vor Beschlag geschützt ist und das bei gegebenenfalls mehrfacher Verwendung hohen hygienischen Ansprüchen genügt.

DE 20 2010 017 255 U1 offenbart ein endoskopisches Instrument mit einer Heizung für das Abschlussfenster.

US 5,536,236 offenbart einen abgedecktes Endoskop-system. JPH03118021 (A) offenbart ein Endoskopsystem mit einer indirekten Heizung für das Abschlussfenster.

US2015238072A1 offenbart ein System und Verfahren zum thermischen Entfrosten.

### Darstellung der Erfindung

Diese Aufgabe wird durch ein Fenstersystem für einen Intraoralscanner gemäß Anspruch 1 gelöst.

Ein optisches Element weist eine Wärmeleitfähigkeit von mehr als 1 W m⁻¹ K⁻¹, vorzugsweise von mehr als 40 W m⁻¹ K⁻¹, auf. Ein Fenster weist eine Scheibe aus einem Kunststoff, einem Glas oder einem Korund auf. Das Fenster ist so an dem optischen Element angeordnet, dass das Fenster einen mittleren Abstand von weniger als 1 mm aufweist. Ein solcher Abstand ermöglicht einen Wärmetransport durch Wärmestrahlung, Konvektion oder Wärmeleitung der eine beschlagmindernde Wirkung erzielt. Der Zwischenraum zwischen dem optischen Element und dem Fenster kann luftleer (Vakuum), mit einem Gas (Luft) gefüllt, aber auch mit einer klebenden oder nicht klebenden Flüssigkeit oder einem Feststoff ausgefüllt sein, sofern der Lichtweg hierdurch nicht signifikant beeinträchtigt wird. Bei ideal planen Flächen ist der Kontakt auf mikroskopischer Ebene vollflächig. Zum jetzigen Stand der Technik ist die Ebenheit der Oberflächen allerdings begrenzt und der Kontakt besteht nur auf Teilflächen bzw. punktuell. Das Fenstersystem weist weiterhin mindestens eine Wärmequelle auf, welche mit dem optischen Element verbunden ist.

Das optische Element ist ein Fenster.

Das Fenstersystem kann so an den Intraoralscanner angebracht werden, dass das optische Element einer Lichtquelle und/oder einem Sensor des Intraoralscanners zugewandt ist. Das Fenster ist dann dem Mundraum eines Patienten zugewandt. Lediglich das Fenster ist einer Kontamination aus dem Mundraum des Patienten ausgesetzt.

Das Material des Fensters ist vorzugsweise ausgewählt aus der Gruppe bestehend aus einem Polycarbonat (PC), einem Cycloolefin-Copolymer (COC, COP), einem Polymethylmethacrylat (PMMA), einem Floatglas, einem Mineralglas oder Gemischen aus diesen Materialien. Es handelt sich hierbei um Materialien, die eine hohe Transparenz aufweisen und den hygienischen Anforderungen an intraoral angewendete medizintechnische Geräte genügen, die dabei aber gleichzeitig so kostengünstig sind, dass sie für den Einmalgebrauch geeignet sind. Da das Fenster lösbar mit dem optischen Element verbunden ist, kann es nach Benutzung des Intraoralscanners durch ein neues Fenster ersetzt werden oder hygienisch wieder aufbereitet werden. Das Fenster kann hierbei sowohl starr als auch als flexible Folie ausgeführt sein.

Alternativ ist das Material des Fensters ein Korund, insbesondere Saphirglas. In dieser Ausführungsform ist das Fenster zum mehrfachen Gebrauch vorgesehen.

Ein eventueller Beschlag des Fenstersystems würde an dem im Mundraum des Patienten zugewandten Fenster erfolgen. Die Erfindung sieht vor, das Fenster zu beheizen, um zu verhindern, dass seine Oberflächentemperatur unter den Taupunkt sinkt. Um die Austauschbarkeit des Fensters zu gewährleisten ist dieses jedoch selbst nicht mit einer Wärmequelle versehen. Stattdessen ist die mindestens eine Wärmequelle am optischen Element angeordnet. Das optische Element besteht hierzu vorzugsweise aus einem Korund. Korund als Material des optischen Elements weist nicht nur eine hohe optische Güte, sondern auch eine hohe Wärmeleitfähigkeit auf. Dadurch kann es die von der mindestens einen Wärmequelle abgegebene Wärme aufnehmen und über seine gesamte Fläche an das Fenster weiterleiten. Dadurch kann die erforderliche Beheizung des Fensters realisiert werden. Während ein Korund, wie insbesondere Saphirglas, für den Einmalgebrauch zu teuer wäre, ermöglicht die Tatsache, dass das optische Element nicht mit dem Mundraum des Patienten in Kontakt gelangt, sondern von diesem durch das Fenster getrennt wird, die Verwendung dieses Materials in dem erfindungsgemäßen Fenstersystem.

Die Wärmequelle ist eingerichtet, um Abwärme des Intraoralscanners an einen Randbereich des optischen Elements weiterzuleiten. Sie produziert also selbst keine Wärme. Hierzu ist die Wärmequelle insbesondere an einer Außenkante des optischen Elements angeordnet. Das Fenstersystem ist so ausgeführt, dass sich kein Teil der Wärmequelle im Lichtweg des Fenstersystems befindet. Die gute Wärmeleitfähigkeit des optischen Elements ermöglicht die von der Wärmequelle abgegebene Wärme vom Rand des optischen Elements zunächst über die gesamte Fläche des optischen Elements und dann an die gesamte Fläche des Fensters weiterzuleiten. Um das Fenster lösbar mit dem optischen Element verbinden zu können und dabei sicherzustellen, dass kein Speichel, Blut oder eine sonstige Flüssigkeit aus dem Mundraum eines Patienten am Fenster vorbei zu dem optischen Element gelangen kann, ist das Fenster in einem Überzieher für den Intraoralscanner angeordnet. Dieser Überzieher, der auch als Hülse bezeichnet werden kann, ist in einer bevorzugten Ausführungsform genauso wie das Fenster als Einwegprodukt ausgeführt. In dieser Ausführungsform ist er fest mit dem Fenster verbunden und kann gemeinsam mit diesem entsorgt werden. Hierzu ist er als Überzieher ausgeführt, der insbesondere aus einem Silikon, aus Latex, aus einer Kunststofffolie oder einem festen Kunststoff, wie insbesondere einem Acrylnitril-Styrol-Acrylat-Copolymer (ASA) oder einem Acrylnitril-Butadien-Styrol-Copolymer (ABS), besteht. Er kann hart oder elastisch ausgeführt sein. Der Überzieher weist einen Fensterbereich auf, in dem das Fenster angeordnet ist. Dieses kann insbesondere mittels Umspritzung oder Verklebung mit dem Kunststoffüberzieher verbunden sein, um eine flüssigkeitsdichte Verbindung zu gewährleisten.

Auch bei einer nicht flüssigkeitsdichten Verbindung zwischen dem Fenster und dem Überzieher kann bei prozessgerechter Anwendung, Reinigung und Desinfektion eine erhöhte Schutzwirkung vor Kreuzkontamination erzielt werden.

In einer anderen bevorzugten Ausführungsform ist der Überzieher mehrfach verwendbar. Hierzu ist er autoklavierbar ausgeführt, sodass er vor seiner erneuten Verwendung sterilisiert werden kann. Geeignete Materialien für einen autoklavierbaren Überzieher können Metalle oder Kunststoffe sein, wobei sowohl harte als auch elastische Kunststoffe eingesetzt werden können. Das Fenster ist in einen Fensterbereich des Überziehers einschiebbar ausgeführt, sodass es nach Benutzung des Überziehers entfernt und entsorgt werden kann. Nach dem Autoklavieren kann der Überzieher dann mit einem neuen Fenster versehen werden. Dieser Überzieher kann aber insbesondere auch mit einem Fenster aus einem Korund ausgestattet sein, das zur mehrfachen Verwendung vorgesehen ist.

In einer nicht erfindungsgemäßen Ausführungsform des Fenstersystems ist die Wärmequelle eine Schicht aus ITO (Indium Tin Oxide), die auf dem optischen Element angeordnet ist. Dabei befindet sie sich in einer Ausführungsform auf der dem Fenster zugewandten Seite des optischen Elements, um Wärme nicht nur an das optische Element, sondern auch direkt an das Fenster abgeben zu können. In einer anderen nicht erfindungsgemäßen Ausführungsform befindet sie sich aus Gründen der elektrischen Sicherheit auf der von dem Fenster abgewandten Seite des optischen Elements. Eine ITO-Schicht ist elektrisch leitfähig und transparent. Wenn sie elektrisch kontaktiert wird, kann sie durch einen Stromfluss Wärme erzeugen.

Um eine Verringerung der Transmission des Fenstersystems durch die Anwesenheit der ITO-Schicht zu verringern, ist es besonders bevorzugt, dass die ITO-Schicht an die Brechzahl des optischen Elements angepasst ist. Eine derartige ITO-Schicht wird als IMITO (Index Matched Indium Tin Oxide) bezeichnet.

Auch eine IMITO-Schicht, die vollflächig auf dem optischen Element angeordnet ist, bewirkt immer noch eine geringfügige Verringerung der Transmission des Fenstersystems gegenüber einem Fenstersystem, das keine derartige Schicht aufweist. Es ist deshalb weiterhin bevorzugt, dass die ITO-Schicht lediglich in einem Randbereich des optischen Elements angeordnet ist. Unter einem Randbereich wird insbesondere ein Bereich des optischen Elements verstanden, dessen Breite maximal 25 Prozent der Breite des optischen Elements entspricht und dessen Länge maximal 25 Prozent der Länge des optischen Elements entspricht. Aufgrund der guten Wärmeleitfähigkeit des optischen Elements genügt es dieses mittels der ITO-Schicht im Randbereich zu erwärmen, damit die Wärme gleichmäßig über das gesamte optische Element verteilt wird und von diesem an die gesamte Fläche des Fensters weitergeleitet wird. Bei dieser Ausführung der ITO-Schicht ist der mittlere Bereich des optischen Elements der als Lichtweg fungiert nicht mit einer ITO-Schicht ausgestattet, sodass die Transmission des Fenstersystems nicht beeinträchtigt wird. Auf diese Weise kann eine Transmission von mehr als 99 Prozent realisiert werden.

Gegenüber einem Fenstersystem, das eine ITO-Schicht aufweist, weist das Fenstersystem gemäß der Erfindung den Vorteil auf, dass es in einfacher Weise gefertigt werden kann. Es werden keine elektrische Kontaktierung und Zuleitung mit der notwendigen Stromzufuhr oder Induktionsspulen zum Erhitzen der ITO-Schicht benötigt. Auch auf eine galvanische Trennung und die Vermeidung von Gefahren für den Patienten durch weitere elektrische Leitungen kann verzichtet werden. Stattdessen genügt es, die Wärmequelle als Bauteil aus einem gut wärmeleitenden Material, wie insbesondere einem Metall auszuführen, das mit wärmeproduzierenden Bauteilen in einem anderen Bereich des Intraoralscanners verbunden ist.

Um eine hohe Qualität der mittels des Intraoralscanners aufgenommenen Daten zu gewährleisten ist es bevorzugt, dass das optische Element auf beiden Seiten seines Lichtwegs, im Falle einer Ausführung als Fenster also auf beiden Seiten seiner Scheibe, jeweils eine Antireflexionsbeschichtung aufweist. Weiterhin ist bevorzugt, dass das Fenster auf beiden Seiten seiner Scheibe ebenfalls eine Antireflexionsbeschichtung aufweist. Normalerweise bringt die Verwendung derartiger Antireflexionsbeschichtungen in intraoral angewendeten medizintechnischen Geräten Probleme mit sich, da sie beim Autoklavieren angegriffen, verschmutzt oder eingetrübt werden und sich dadurch die optische Güte der Beschichtung verringert. Die Beschichtungen auf dem optischen Element sind in dem Fenstersystem jedoch durch das Fenster geschützt und müssen daher keinem Autoklaviervorgang zu ihrer Sterilisierung unterworfen werden. Des Weiteren ist das optische Element vor Kratzern geschützt, welche ebenso eine Beeinträchtigung der 3D Messung und/oder eine Beeinträchtigung der Datenqualität verursachen können.

Da das Fenster als Einwegprodukt ausgeführt werden kann, können die darauf aufgebrachten Antireflexionsschichten gemeinsam mit dem Fenster entsorgt werden.

Ein Intraoralscanner, der ein Fenstersystem aufweist, in dem das Fenster in einem Überzieher für den Intraoralscanner angeordnet ist, ist so ausgeführt, dass das optische Element und die mindestens eine Wärmequelle mit dem Intraoralscanner verbunden sind. Der Überzieher ist so an dem Intraoralscanner anordenbar, dass das Fenster in einem mittleren Abstand von weniger als 1 mm von dem optischen Element anordenbar ist.

### Kurze Beschreibung der Zeichnungen

Ausführungsbeispiele der Erfindung sind in Zeichnungen dargestellt und in der nachfolgenden Beschreibung näher erläutert.
Fig. 1 zeigt eine isometrische Darstellung eines Intraoralscanners gemäß einem Ausführungsbeispiel der Erfindung.
Fig. 2 zeigt eine Explosionsdarstellung eines Fenstersystems gemäß einer nicht erfindungsgemässen Ausführungsform.
Fig. 3 zeigt eine Aufsicht auf ein Fenstersystem gemäß einer nicht erfindungsgemässen Ausführungsform.
Fig. 4 zeigt eine Explosionsdarstellung eines Fenstersystems gemäß einem Ausführungsbeispiel der Erfindung.

### Ausführungsbeispiele

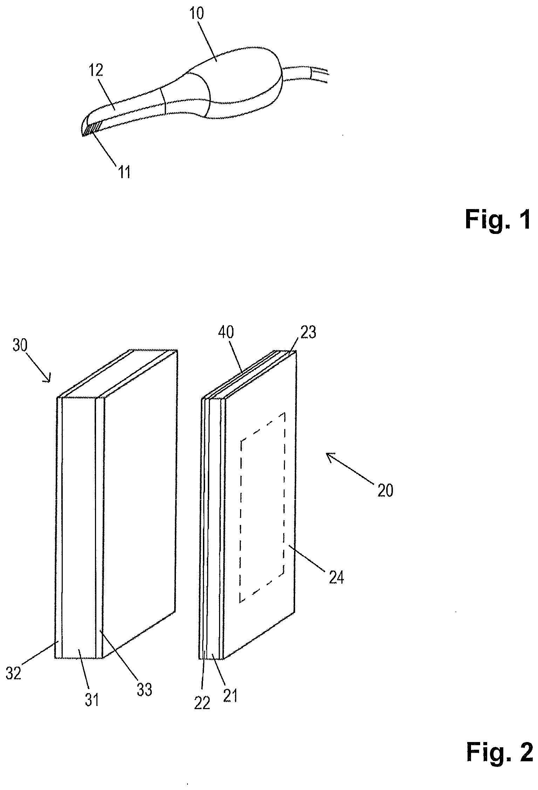

In einem in Fig. 1 dargestellten Ausführungsbeispiel der Erfindung weist ein Intraoralscanner 10 einen Fensterbereich 11 auf, in dem ein Fenstersystem angeordnet ist. Das Fenstersystem dient als Lichtweg für einen Lichtstrahl, der von einer im Intraoralscanner 10 angeordneten Lichtquelle ausgesandt wird und als Lichtweg für einen von einem Zahn im Mundraum eines Patienten reflektierten Lichtstrahl, der auf einen Sensor im Inneren des Intraoralscanners 10 zurückreflektiert wird. Der Intraoralscanner 10 weist in dem Bereich, in dem er in den Mundraum eines Patienten eingeführt wird, einen Überzieher 12. Ein Fenster des Fenstersystems ist ein Teil des Überziehers 12, während ein anderes optisches Element des Fenstersystems ein Teil des Intraoralscanners 10 ist.

In einem ersten Ausführungsbeispiel des Überziehers besteht dieser aus einem elastischen Silikon, mit welchem ein Fenster des Fenstersystems im Fensterbereich 11 umspritzt ist.

In einem zweiten Ausführungsbeispiel des Überziehers 12 besteht dieser aus Stahl. Er weist im Fensterbereich 11 einen Einschubrahmen auf, in den ein Fenster des Fenstersystems eingesetzt ist.

In einem dritten Ausführungsbeispiel des Überziehers 12 besteht dieser aus ASA, in welches das Fenster eingesetzt ist.

Fig. 2 zeigt ein erstes Fenster 20 als optisches Element und ein zweites Fenster 30 eines Fenstersystems gemäß einem nicht erfindungsgemäßen Ausführungsbeispiel des Fenstersystems. Das erste Fenster 20 ist ein Teil des Intraoralscanners 10, während das zweite Fenster 30 ein Teil des Überziehers 12 ist. Das erste Fenster 20 weist eine Scheibe 21 auf, die aus Saphirglas mit einer Wärmeleitfähigkeit von 41,9 W m⁻¹ K⁻¹ besteht. Sie ist auf beiden Seiten jeweils vollflächig mit einer Antireflexionsbeschichtung 22, 23 versehen. In einem Randbereich 24, der in Fig. 2 mit einer gestrichelten Linie vom Rest des ersten Fensters 20 abgeteilt ist, weist es auf seiner dem zweiten Fenster 30 zugewandten Seite eine Wärmequelle 40 in Form einer ITO-Schicht auf. Diese ist elektrisch so kontaktiert, dass sie durch einen elektrischen Stromfluss oder durch Induktion erhitzt werden kann. Das zweite Fenster 30 weist in diesem Ausführungsbeispiel eine Scheibe 31 aus Polycarbonat auf, die auf beiden Seiten jeweils vollflächig mit einer Antireflexionsbeschichtung 32, 33 beschichtet ist. Wenn der Überzieher 12 über den Intraoralscanner 10 gezogen ist, dann liegt das zweite Fenster 30 mit einer seiner Antireflexionsschichten 33 so auf dem ersten Fenster 20 auf, dass es im Randbereich 24 die Wärmequelle 40 kontaktiert und in der Mitte des ersten Fensters 20 eine von dessen Antireflexionsschichten 22 kontaktiert. Der Randbereich 24 ist nicht Teil des Lichtweges, sodass die Wärmequelle 40 die Transmission des Lichtweges nicht beeinträchtigt. Wenn der Intraoralscanner 10 sich in Betrieb befindet, ist die Wärmequelle 40 eingeschaltet und beheizt sowohl das erste Fenster 20 als auch das zweite Fenster 30 in ihrem jeweiligen Randbereich. Während sich die Wärme im zweiten Fenster 30 aufgrund der schlechten Wärmeleitfähigkeit von Polycarbonat zunächst nicht gleichmäßig verteilen kann, bewirkt die gute Wärmeleitfähigkeit des Saphirglases eine gleichmäßige Erhitzung des ersten Fensters 20. Dieses gibt die Wärme über seine gesamte Fläche an das zweite Fenster 30 weiter, sodass nach kurzer Zeit auch dieses eine gleichmäßige Temperaturverteilung aufweist.

In einem zweiten nicht erfindungsgemäßen Ausführungsbeispiel des Fenstersystems, das nicht dargestellt ist, ist die Wärmequelle 40 nicht auf der dem zweiten Fenster 30 zugewandten Seite, sondern auf der dem zweiten Fenster 30 abgewandten Seite angeordnet.

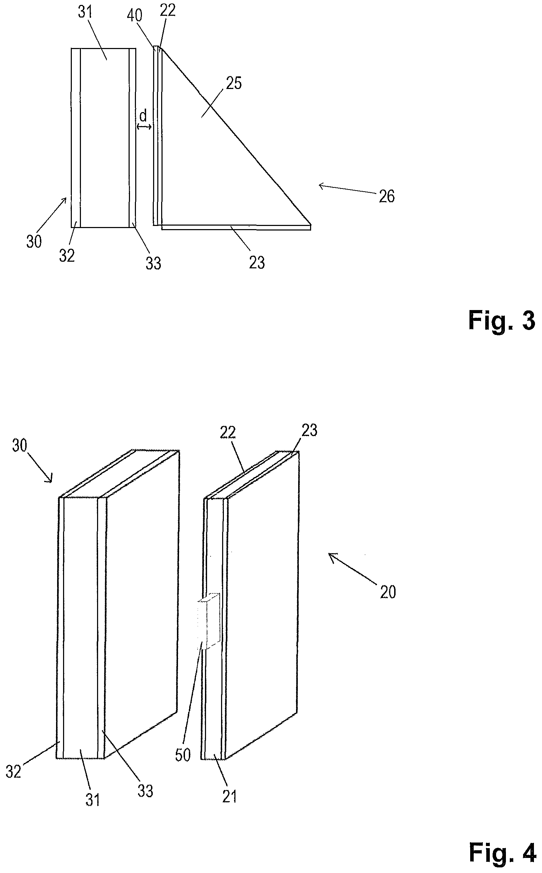

Ein drittes nicht erfindungsgemäßes Ausführungsbeispiel des Fenstersystems ist in Fig. 3 dargestellt. Diese unterscheidet sich von dem Fenstersystem gemäß dem ersten Ausführungsbeispiel darin, dass als optisches Element als Prisma 26 ausgeführt ist. Dieses weist ein Prismenelement 25 auf, das auf beiden Seiten seines Lichtweges jeweils vollflächig mit einer Antireflexionsbeschichtung 22, 23 versehen. Auf seiner dem zweiten Fenster 30 zugewandten Seite weist es eine Wärmequelle 40 in Form einer ITO-Schicht auf. Der mittlere Abstand d zwischen dem Prisma 26 und dem zweiten Fenster 30 beträgt 0,2 mm.

Ein Ausführungsbeispiel des erfindungsgemäßen Fenstersystems ist in Fig. 4 dargestellt. Dieses unterscheidet sich von dem Fenstersystem gemäß dem ersten Ausführungsbeispiel darin, dass es keine ITO-Schicht als Wärmequelle 40 aufweist. Stattdessen, ist an den Kanten des ersten Fensters 20 jeweils eine Wärmequelle 50 angeordnet, die so mit wärmeerzeugten Komponenten des Intraoralscanners 10 verbunden ist, dass deren Abwärme an den Rand des ersten Fensters 20 geleitet wird. Auch wenn die Wärmequelle 50 in der Fig. 4 aus Gründen der besseren Darstellbarkeit so dargestellt ist, dass sie nur ein Teil einer Kante des ersten Fensters 40 kontaktiert, ist sie jedoch tatsächlich als Rahmen ausgeführt, der vollständig um alle vier Kanten des ersten Fensters 20 umläuft. Im Betrieb des Intraoralscanners 10 erwärmen sich verschiedene seiner Komponenten, wie beispielsweise seine Lichtquelle, und leiten einen Teil ihrer Abwärme über die Wärmequelle 50 an die Ränder des ersten Fensters 20 weiter. Aufgrund von dessen guter Wärmeleitfähigkeit verteilt sich die Wärme gleichmäßig über das erste Fenster 20 und wird von diesem dann an die gesamte Oberfläche des zweiten Fensters 30 weitergegeben.

Nach Anwendung des Intraoralscanners 10 wird der Überzieher 12 von diesem entfernt. Wenn das zweite Fenster 30 fest mit dem Überzieher 12 verbunden ist, wird es gemeinsam mit diesem entsorgt. Andernfalls wird es aus dem Rahmen des Überziehers 12 entfernt, der Überzieher 12 wird autoklaviert und anschließend mit einem neuen zweiten Fenster 30 versehen. Auf diese Weise wird gewährleistet, dass beim nächsten Gebrauch des Intraoralscanners 10 der gesamte Bereich, der mit dem Mundraum eines Patienten in Kontakt kommt, sauber und steril ist.

Wenn das zweite Fenster nicht flüssigkeitsdicht mit dem Überzieher verbunden ist, erfordert die Verwendung am Patienten unter Umständen eine festgelegten Reinigungs- und Desinfektions- und/oder Sterilisationsprozedur des Überziehers 12 um die Wahrscheinlichkeit der Kreuzkontamination zu reduzieren. In solch einem Fall wird nach Anwendung des Intraoralscanners 10 der Überzieher 12 von diesem entfernt und das zweite Fenster 30 entsorgt.

Wenn das zweite Fenster 30 fest aber nicht flüssigkeitsdicht mit dem Überzieher 12 verbunden ist, wird es gemeinsam mit diesem entsorgt und der Intraoralscanner muss unter Umständen zusätzlich gereinigt und/oder desinfiziert und/oder sterilisiert werden. Dies kann beispielsweise durch Wischdesinfektion erfolgen. Für den nächsten Patienten muss ein neuer, sauberer Überzieher 12 auf die Intraoralkamera 10 gezogen werden.

## Patentansprüche

1. Fenstersystem für einen Intraoralscanner (10), aufweisend
- ein optisches Element (20) als erstes Fenster () mit einer Wärmeleitfähigkeit von mehr als 1 W m⁻¹ K⁻¹ aufweist,
- ein zweites Fenster (30), welches eine Scheibe (31) aus einem Kunststoff, einem Glas oder einem Korund aufweist, und welches von dem optischen Element (20) einen mittleren Abstand (d) von weniger als 1 mm aufweist, und
- mindestens eine Wärmequelle (50) aufweist, welche mit dem optischen Element (20) verbunden ist, die Wärmequelle (50) als Wärmeübertragungssystem ausgeführt ist, um Abwärme des Intraoralscanners (10) an einen Randbereich des optischen Elements (20) weiterzuleiten,
- wobei das zweite Fenster (30) in einem entfernbaren Überzieher (12) für den Intraoralscanner (10) angeordnet ist,
wobei das Fenstersystem keine ITO-Schicht als Wärmequelle (40) aufweist, stattdessen, ist die Wärmequelle (50) jeweils an den Kanten des ersten Fensters (20) angeordnet, die so mit wärmeerzeugten Komponenten des Intraoralscanners (10) verbunden ist, dass deren Abwärme an den Rand des ersten Fensters (20) geleitet wird, wobei die Wärmequelle (50) als Rahmen ausgeführt ist, der vollständig um alle vier Kanten des ersten Fensters (20) umläuft, wobei sich im Betrieb des Intraoralscanners (10) verschiedene seiner Komponenten erwärmen, wie beispielsweise seine Lichtquelle, und einen Teil ihrer Abwärme über die Wärmequelle (50) an die Ränder des ersten Fensters (20) weiterleiten, wobei
sich Aufgrund von dessen Wärmeleitfähigkeit die Wärme gleichmäßig über das erste Fenster (20) verteilt und von diesem dann an die gesamte Oberfläche des zweiten Fensters (30) weitergegeben wird.

2. Fenstersystem nach Anspruch 1, **dadurch gekennzeichnet, dass** das optische Element (20, 26) eine Wärmeleitfähigkeit von mehr als 40 W m⁻¹ K⁻¹ aufweist.

3. Fenstersystem nach Anspruch 1 oder 2, **dadurch gekennzeichnet, dass** das optische Element (20, 26) aus einem Korund besteht.

4. Fenstersystem nach einem der Ansprüche 1 bis 3, **dadurch gekennzeichnet, dass** der mittleren Abstand (d) zwischen dem optischen Element (20, 26) und dem zweiten Fenster weniger als 0,5 mm beträgt.

5. Fenstersystem nach einem der Ansprüche 1 bis 4, **dadurch gekennzeichnet, dass** das Material des zweiten Fensters (30) ausgewählt ist aus der Gruppe, bestehend aus einem Polycarbonat, einem Cycloolefin-Copolymer, einem Polyacrylmethacrylat, einem Floatglas, einem Mineralglas, einem Korund oder Gemischen daraus.

6. Fenstersystem nach einem der Ansprüche 1 bis 4, **dadurch gekennzeichnet, dass** zweite Fenster (30) aus einem Korund besteht.

7. Fenstersystem nach einem der Ansprüche 1 bis 6, **dadurch gekennzeichnet, dass** das optische Element (20, 26) auf beiden Seiten seines Lichtwegs jeweils eine Antireflexionsbeschichtung (22, 23) aufweist.

8. Fenstersystem nach einem der Ansprüche 1 bis 7, **dadurch gekennzeichnet, dass** das zweite Fenster (30) auf beiden Seiten seiner Scheibe (31) jeweils eine Antireflexionsbeschichtung (32, 33) aufweist.

9. Intraoralscanner (10) aufweisend ein Fenstersystem nach einem der Ansprüche 1 bis 8, wobei das optische Element (20) und die mindestens eine Wärmequelle (40, 50) mit dem Intraoralscanner (10) verbunden sind und der Überzieher (12) so an dem Intraoralscanner (10) entfernbar angeordnet ist, dass das zweite Fenster (30) an dem optischen Element (20) in einem mittleren Abstand von weniger als 1 mm angeordnet ist.

## Claims

1. Window system for an intraoral scanner (10) having
- an optical element (20) as a first window () which has a thermal conductivity of more than 1 W m⁻¹ K⁻¹,
- a second window (30) which has a pane (31) made of a plastic, a glass or a corundum and which has an average distance (d) of less than 1 mm from the optical element (20), and
- having at least one heat source (50) connected to the optical element (20), the heat source (50) being designed as a heat transfer system to transfer waste heat from the intraoral scanner (10) to an edge region of the optical element (20),
- wherein the second window (30) is arranged in a removable cover (12) for the intraoral scanner (10),
wherein the window system does not have an ITO layer as a heat source (40), and instead, the heat source (50) is respectively arranged at the edges of the first window (20), which is connected to heat-generated components of the intraoral scanner (10) such that their waste heat is conducted to the edge of the first window (20), wherein the heat source (50) is designed as a frame which runs completely around all four edges of the first window (20), wherein, during operation of the intraoral scanner (10), various of its components heat up, such as its light source, and transfer part of their waste heat via the heat source (50) to the edges of the first window (20),
wherein, due to its thermal conductivity, the heat is distributed evenly over the first window (20) and is then transferred by the latter to the entire surface of the second window (30).

2. Window system according to claim 1, **characterised in that** the optical element (20, 26) has a thermal conductivity of more than 40 W m⁻¹ K⁻¹.

3. Window system according to claim 1 or 2, **characterised in that** the optical element (20, 26) consists of a corundum.

4. Window system according to one of claims 1 to 3, **characterised in that** the average distance (d) between the optical element (20, 26) and the second window is less than 0.5 mm.

5. Window system according to one of claims 1 to 4, **characterised in that** the material of the second window (30) is selected from the group consisting of a polycarbonate, a cyclic olefin copolymer, a polyacrylic methacrylate, a float glass, a mineral glass, a corundum, or mixtures thereof.

6. Window system according to one of claims 1 to 4, **characterised in that** the second window (30) consists of a corundum.

7. Window system according to one of claims 1 to 6, **characterised in that** the optical element (20, 26) has an anti-reflection coating (22, 23) on each of the two sides of its light path.

8. Window system according to one of claims 1 to 7, **characterised in that** the second window (30) has an anti-reflection coating (32, 33) on each of the two sides of its pane (31).

9. Intraoral scanner (10) having a window system according to one of claims 1 to 8, wherein the optical element (20) and the at least one heat source (40, 50) are connected to the intraoral scanner (10) and the cover (12) is arranged removably on the intraoral scanner (10) such that the second window (30) is arranged on the optical element (20) at an average distance of less than 1 mm.

## Revendications

1. Système de fenêtre pour un scanner intra-oral (10), comportant
- un élément optique (20) en tant que première fenêtre () présentant une conductivité thermique supérieure à 1 W m⁻¹ K⁻¹,
- une seconde fenêtre (30) qui présente une plaque (31) en matière plastique, en verre ou en corindon et qui présente, par rapport à l'élément optique (20), une distance moyenne (d) inférieure à 1 mm, et
- au moins une source de chaleur (50) qui est reliée à l'élément optique (20), la source de chaleur (50) étant conçue comme un système de transfert de chaleur pour transmettre la chaleur dissipée du scanner intra-oral (10) à une zone périphérique de l'élément optique (20),
- la seconde fenêtre (30) étant disposée dans un recouvrement amovible (12) pour le scanner intra-oral (10),
dans lequel le système de fenêtre ne présente aucune couche d'ITO comme source de chaleur (40), la source de chaleur (50) étant au contraire respectivement disposée sur les arêtes de la première fenêtre (20), ladite source de chaleur étant ainsi reliée à des composants générateurs de chaleur du scanner intra-oral (10) de sorte que leur chaleur dissipée est dirigée vers le bord de la première fenêtre (20), la source de chaleur (50) étant conçue sous la forme d'un cadre qui entoure complètement les quatre arêtes de la première fenêtre (20), plusieurs des composants, tels que par exemple la source lumineuse, se réchauffant pendant le fonctionnement du scanner intra-oral (10) et transmettant une partie de leur chaleur dissipée aux bords de la première fenêtre (20) par l'intermédiaire de la source de chaleur (50), dans lequel
en raison de la conductivité thermique de la première fenêtre (20), la chaleur se répartit uniformément sur celle-ci puis est transmise par celle-ci à toute la surface de la seconde fenêtre (30).

2. Système de fenêtre selon la revendication 1, **caractérisé en ce que** l'élément optique (20, 26) présente une conductivité thermique supérieure à 40 W m⁻¹ K⁻¹.

3. Système de fenêtre selon l'une des revendications 1 ou 2, **caractérisé en ce que** l'élément optique (20, 26) est constitué de corindon.

4. Système de fenêtre selon l'une quelconque des revendications 1 à 3, **caractérisé en ce que** la distance moyenne (d) entre l'élément optique (20, 26) et la seconde fenêtre est inférieure à 0,5 mm.

5. Système de fenêtre selon l'une quelconque des revendications 1 à 4, **caractérisé en ce que** le matériau de la seconde fenêtre (30) est choisi dans le groupe constitué de polycarbonate, de copolymère de cyclooléfine, de polyméthacrylate de méthyle, de verre flotté, de verre minéral, de corindon ou de mélanges de ceux-ci.

6. Système de fenêtre selon l'une quelconque des revendications 1 à 4, **caractérisé en ce que** la seconde fenêtre (30) est constituée de corindon.

7. Système de fenêtre selon l'une quelconque des revendications 1 à 6, **caractérisé en ce que** l'élément optique (20, 26) présente respectivement un revêtement antireflet (22, 23) des deux côtés de son chemin optique.

8. Système de fenêtre selon l'une quelconque des revendications 1 à 7, **caractérisé en ce que** la seconde fenêtre (30) présente respectivement un revêtement antireflet (32, 33) des deux côtés de sa plaque (31).

9. Scanner intra-oral (10) comportant un système de fenêtre selon l'une quelconque des revendications 1 à 8, dans lequel l'élément optique (20) et l'au moins une source de chaleur (40, 50) sont reliés au scanner intra-oral (10) et le recouvrement (12) est disposé de manière amovible sur le scanner intra-oral (10) de sorte que la seconde fenêtre (30) est disposée, par rapport à l'élément optique (20), à une distance moyenne inférieure à 1 mm.
